# EUROPÄISCHE PATENTSCHRIFT

(11) **EP 0 521 305 B1**
(45) Veröffentlichungstag und Bekanntmachung des Hinweises auf die Patenterteilung: **29.12.1999**
(21) Anmeldenummer: 92109292.0
(22) Anmeldetag: 02.06.1992
(51) Int. Cl.: C12N 9/78, C12N 9/96, C12P 13/10

(54) **Stabilisierte metallionenaktivierte L-Arginase (L-Arginin Amidinohydrolase; E.C. 3.5.3.1.)**
Stabilized metal ion activated L-Arginase (L-Arginine amidinohydrolase; E.C. 3.5.3.1.)
L-Arginase stabilisée et activée par un ion métallique (L-Arginine amidinohydrolase; E.C. 3.5.3.1.)

(30) Priorität: 10.06.1991 DE 4119029
(43) Veröffentlichungstag der Anmeldung: 07.01.1993
(73) Patentinhaber: Degussa-Hüls Aktiengesellschaft, 60287 Frankfurt am Main (DE)
(72) Erfinder: Bommarius, Andreas, Dr., W-6000 Frankfurt 1 (DE); Drauz, Karlheinz, Dr., W-6463 Freigericht (DE); Makryaleas, Kyriakos, Dr., W-6463 Freigericht 1 (DE)

(56) Entgegenhaltungen:
- EP-A- 0 464 325
- BIOMEDICA BIOCHIMICA ACTA Bd. 50, Nr. 10, 1991, BERLIN, GERMANY Seiten 249 - 255 A.S. BOMMARIUS ET AL. 'An enzymatic route to L-ornithine from L-arginine. Activation and stabilization studies on L-arginase'
- DATABASE WPI Week 7412, Derwent Publications Ltd., London, GB; AN 74-21642V (12)
- BIOTECHNOLOGY TECHNIQUES Bd. 4, Nr. 2, 1990, GB Seiten 133 - 136 N.B. PATIL ET AL. 'A simple and rapid high recovery protocol for the purification of arginase'
- EUROPEAN JOURNAL OF BIOCHEMISTRY Bd. 127, Nr. 2, Oktober 1982, BERLIN, G Seiten 237 - 243 J.-P. BOUTIN 'Purification properties and subunit structure of arginase from Iris bulbs'
- BIOCHIM. BIOPHYS. ACTA Bd. 527, Nr. 1, 10. November 1978, AMSTERDAM, NL Seiten 1 - 7 N. CARVAJAL ET AL. 'Subunit interactions and immobilized dimers of human liver arginase'
- BIOINORGANIC CHEMISTRY Bd. 6, Nr. 2, 1976, Seiten 133 - 142 M. MUNAKATA ET AL. 'The role of metal ions in the activation of arginase'

## Beschreibung

Die Erfindung betrifft einen stabilisierten Arginaseansatz, ein Verfahren zur enzymatischen Umwandlung von Arginin in Ornithin in wässriger Lösung und einen Arginase-Kit hierfür.

Arginase (L-Arginase, L-Arginin Amidinohydrolase; E.C. 3.5.3.1.) ist ein seit über 50 Jahren bekanntes Enzym, das die enzymatische Herstellung von L-Ornithin aus L-Arginin katalysiert. In vivo findet diese Katalyse in der Leber bei Säugetieren im letzten Schritt des Harnstoffzyklus statt, wobei durch Hydrolyse aus (L-Arginin 2-Amino-5-guanidinopentansäure) Harnstoff und L-Ornithin (2,5-Diaminopentansäure) gebildet wird. Das Enzym kann entsprechend aus Säugetierleber, z. B. Kalbsleber, gewonnen werden, es kommt aber auch im Pflanzenreich sowie in mehreren Mikroorganismen vor.

Die Arginase hat ein Molekulargewicht von ca. 138 000 und besteht aus 4 identischen Untereinheiten. Als typischer Aktivator kann Mn²⁺, ferner auch Co²⁺ und Ni²⁺ zugesetzt werden.

L-Ornithin ist eine im Körper von Säugetieren vorkommende, aber nicht beim Anabolismus entstehende und daher nicht in Proteinen eingebaute, d. h. natürliche aber nichtproteinogene, Aminosäure.

L-Ornithin kann L-Arginin, eine bei Säuglingen und Kindern essentielle Aminosäure, in allen Funktionen ersetzen. Da die Salze des L-Ornithin eine geringere Belastung mit Harnstoff für den Organismus mit sich bringen und teilweise ein besseres Löslichkeitsverhalten halten zeigen als L-Arginin, besitzt L-Ornithin ein erhebliches kommerzielles Potential. Ein Mangel an Arginin bzw. Ornithin kann zu Schädigungen bis hin zum Tod z. B. durch überhöhten Ammoniakspiegel wegen hoher Aminosäureaufnahme nach einer Periode des Fastens oder einer Unterernährung führen.

Arginase ist als diagnostisches Enzym seit langem in Gebrauch. Zur Herstellung von L-Ornithin aus L-Arginin im technischen Maßstab konnte dieses Enzym jedoch bis heute nicht verwendet werden, da es unter Reaktionsbedingungen nur eine sehr geringe Stabilität hat und entsprechend aus einem Enzymansatz gar nicht oder nur in geringem Ausmaße wiedergewonnen werden kann. Verbunden mit den hohen Enzymkosten ist daher die enzymatische Produktion von L-Ornithin aus L-Arginin im technischen Maßstab nicht lohnend.

Als Syntheseverfahren für L-Ornithin und seine Salze kommt deshalb neben dem enzymatischen Verfahren großtechnisch nur die Fermentation aus Glucose mittels der Stämme Brevibacterium, Corynebacterium und Arthrobacter, sowie die chemische Hydrolyse von L-Arginin in Betracht. Die chemische Hydrolyse führt jedoch zu vielen Nebenprodukten, so zum Beispiel zur partiellen Hydrolyse zu L-Citrullin (2-Amino-5-ureido-pentansäure) oder zur Racemisierung von L-Arginin oder L-Ornithin. Die Fermentation zu L-Ornithin ist erst in hohen Tonnagen wirtschaftlich.

Obwohl Arginase eine zufriedenstellende Aktivität und Selektivität für die Hydrolyse von L-Arginin zu L-Ornithin besitzt, ist die Stabilität des Enzyms für einen technischen Einsatz unzureichend. Um eine gute Aktivität zu erhalten, ist der Zusatz von zweiwertigen Manganionen neben dem Enzym zur Reaktionslösung notwendig. Beim gewöhnlich eingestellten pH-Wert der Reaktion von 9,5, der dem in der Literatur publizierten Aktivitätsoptimum der Arginase entspricht, fällt jedoch wegen der Oxidation des zweiwertigen zum vierwertigen Mangan häufig nach kurzer Zeit Braunstein (Mangandioxid) aus. Hiermit ist gleichzeitig eine Desaktivierung der Arginase verbunden (M. Munakata et al., Bioinorganic Chemistry 1976, 6, 133 - 42; V. Rossi et al., Int. J. Peptide Protein Res. 1983, 22, 239 - 50 ).

N.B. Patil et al., Biotechnology Techniques (1990), 4(2), 133-136 offenbart ein Verfahren zur Isolierung von Arginase, wobei dieses Verfahren aus sechs verschiedenen Schritten besteht. Das Dokument beschreibt auch, daß alle diese Schritte in Anwesenheit von einem Reduktionsmittel (z-Mercaptoethanol oder DTT) durchgeführt werden. Als Endprodukt wird eine wässeriger lösung mit Reduktionsmittel und homogener Arginase erhalten.

Aufgabe der Erfindung ist eine stabilisierte Form eines Arginaseansatzes, der auch in einem breiteren pH-Bereich einsetzbar sein soll, ohne dabei übermässig desaktiviert zu werden. Zudem soll die Arginase nach der Reaktion gegebenenfalls zur Wiederverwertung abtrennbar sein. Aufgabe der Erfindung ist auch ein entsprechender Arginase-Kit und ein Verfahren zur enzymatischen Umwandlung von L-Arginin in L-Ornithin in Lösung, beim dem die Arginase gegebenenfalls ebenfalls wiederverwertbar sein soll.

Diese Aufgabe wird hinsichtlich des stabilisierten Arginaseansatzes durch die kennzeichnenden Merkmale des Anspruchs 1 gelöst.

Ein üblicher Arginaseansatz enthält in Wasser gelöst das Enzym Arginase, ein Substrat, das durch das Enzym umgesetzt werden soll und gegebenenfalls Mn²⁺.

Der erfindungsgemäße Arginase-Ansatz, der Arginase-Kit und das Verfahren zeichnen sich durch die Zugabe von Ascorbinsäure als Reduktionsmittels aus, die in mindestens 10-facher molarer Konzentration gegenüber der Arginase vorliegen soll.

Aus Konzentrations- und Kostengründen kann als obere Grenze des Überschusses eine gegenüber dem Enzym 10⁶-fache molare Menge des Reduktionsmittel angesetzt werden. Vorteilhaft sind Gaben des Reduktionsmittel in 10² bis 10⁴-fachem molaren Überschuß gegenüber der Arginase, wobei das Reduktionsmittel vorteilhaft in einer Konzentration von 10⁻⁷ - 10⁻¹ mol/l, vorzugsweise 10⁻⁵ - 10⁻³ mol/l vorliegt. Als günstiges Reduktionsmittel hat sich Ascorbinsäure (L-Ascorbinsäure, Vitamin C) erwiesen.

Die Arginasereaktion findet zweckmäßigerweise bei einer 10⁻⁸ bis 10⁻⁵ molaren Konzentration (mol/l) an Arginase statt, dabei ist es für eine gute Arginaseaktivität günstig, wenn Mn²⁺-Ionen in 10 bis 10⁶-fachem molaren Überschuß gegenüber der Arginase vorliegen. Das Lösungsmittel ist vorzugsweise rein wässrig. Vorteilhaft wird das Enzym, z. B. Arginase aus Kalbsleber, zwischen 1000 und 10 000 U/l eingesetzt; Mn²⁺ wird vorteilhaft zwischen 10⁻⁴ und 10⁻² mol/l, bevorzugt als Mangansulfat zugesetzt. Hierbei wurde weiterhin ermittelt, daß bei einem stöchiometrischen Unterschuß des Reduktionsmittels gegenüber den Mn²⁺-Ionen, insbesondere bei einem Mol-Verhältnis zwischen Reduktionsmitteln zu Mn²⁺-Ionen von 0,01 - 0,9, eine besonders gute Stabilität der Arginase erreicht wird. Am größten ist dabei die Stabilität, wenn das Mol-Verhältnis Reduktionsmittel zu Mn²⁺-Ionen zwischen 0,1 und 0,5 liegt. Diese Verhältnisse sind besonders dann angezeigt, wenn die Arginasereaktion bei einem pH von 8,5 - 10,5 gestartet wird. Der pH von 8,5 - 10,5 kann durch Zugabe von Säuren (z. 8. H₂SO₄, HCl) zur Arginin-Lösung eingestellt werden, Säure, Arginin und/oder Ornithin bilden dabei das Puffersystem.

Dem Arginaseansatz können als Substrat vorteilhaft 0,1 bis 2,0 mol/l Arginin zugegeben werden, wobei eine gesättigte Lösung mit Arginin als Bodensatz vorliegen kann. Bei pufferfreien Ansätzen kann auch das gebildete Ornithin ausfallen. Für eine gute Arginaseaktivität und hohe Wiedergewinnungsraten an Arginase haben sich insbesondere Argininkonzentrationen zwischen 0,5 bis 1,5 mol/l, am besten bis 1,0 mol/l bewährt. Die Argininlösung braucht dabei nicht gepuffert zu werden, d. h. der Anfangs-pH-Wert der Lösung darf bei über 10,5 bis 11,5, vorzugsweise 11 bis 11,5 liegen. Dieser Wert wird dabei in erster Linie durch den Eigen-pH-Wert des Arginins bestimmt. Im Laufe der Reaktion sinkt dann dieser pH auf ca. 9,5, bedingt durch die Umsetzung Arginin zu Ornithin. Insbesondere bei diesem Anfangs-pH sollte die Konzentration des Reduktionsmittels mindestens 10⁻⁵ mol/l betragen.

Es hat sich dabei gezeigt, daß im Gegensatz zum in der Literatur propagierten pH-Optimum von 9,5 das Enzym bei pH-Werten bis 11,5 mindestens zwei Tage aktiv ist und sogar die größte Aktivität zeigt.

Das erfindungsgemäße Verfahren zur enzymatischen Gewinnung von Ornithin aus Arginin wird also durchgeführt, indem man Arginin (ggf. mit Bodensatz) in wässriger Lösung mit einer Arginase und ggf. in Anwesenheit von Mn²⁺ und ggf. einem Puffersystem umsetzt, wobei in der Lösung ein Reduktionsmittel in einer mindestens 10-fachen molaren Menge gegenüber der Arginase vorliegt. Für die bevorzugten Maßnahmen des Verfahrens gelten prinzipiell die besonderen Ausführungen des Arginaseansatzes entsprechend.

Die Reaktion sollte in ruhender, d. h. nicht gerührter oder geschüttelter, Lösung erfolgen, da die in bewegter Lösung auftretenden Scherkräfte das Enzym desaktivieren können. Die Reaktionszeit beträgt üblicherweise 24 - 72 Stunden, wobei der Umsatz des L-Arginins vollständig ist. Insbesondere bei ungepufferten Ansätzen kann die Reaktion an Hand der Abnahme des pH-Wertes verfolgt werden, der pH sinkt gegen Ende auf ca. 9,5, dem Eigen-pH des entstehenden L-Ornithins.

Die erfindungsgemäße Zugabe des Reduktionsmittels erlaubt also Arginaseansätze in einem wesentlich breiteren pH-Bereich als bei den Arginaseansätzen ohne Reduktionsmittel, bei denen der pH nahe bei 9,5 sein sollte. Dies hat den Vorteil, daß mit höheren Argininanfangskonzentrationen und ohne zusätzlichem Puffersystem gearbeitet werden kann, wobei trotzdem hohe Wiedergewinnungsraten des Arginins mehrfache Ansätze mit einer Enzymmenge erlauben. Arbeiten in pufferfreien oder -armen Systemen hat dabei den Vorteil der Verringerung der Salzfracht im Prozess. Gegenüber den bisherigen Arginaseansätzen wird durch den Zusatz des Reduktionsmittels die Desaktivierung der Arginase auf weniger als ein Zehntel der Werte ohne Reduktionsmittelzusatz herabgesetzt, d. h. die Arginase wird durch die erfindungsgemäße Reduktionsmittelzugabe um mindestens den Faktor 10 stabilisiert.

Die Stabilisierung läßt sich als Enzymverbrauchszahl ausdrücken, d. h. als Verbrauch an Units Arginase pro kg erzeugten Ornithin bei Rezyklierung des Enzyms aus jedem Ansatz. Diese Verbrauchszahl beträgt bei einem Arginaseansatz
- mit Pumpbewegung ohne Reduktionsmittel 5890,
- ohne Pumpbewegung ohne Reduktionsmittel 5000, und
- ohne Pumpbewegung mit Reduktionsmittel 270.

Bedingungen wie Beispiel 1.

Diese Stabilisierung ermöglicht eine wirtschaftliche Rückgewinnung der Arginase nach der Reaktion, die praktisch vollständig ablaufen kann. Obwohl laut Literatur das L-Ornithin Arginase hemmt, ist der Einfluß bei der erfindungsgemäßen Reaktion nicht entscheidend. Zur Rückgewinnung wird das Enzym aus dem Arginaseansatz mittels Ultrafiltration, vorzugsweise mit einer Kapillarultrafiltrationsdurchlaufkartusche (z. B. Romicon, Trenngrenze 10 000 Dalton), von den niedermolekularen Bestandteilen zumindest weitgehend getrennt. Bei der Kartusche fließt der Ansatz mit ausgefallenem Mangandioxid durch die Kapillaren, wobei der Großteil der Lösung mit den niedermolekularen gelösten Bestandteilen die Poren der Kapillaren durchdringt und das Enzym zusammen mit dem ausgefallenen Braunstein in einem geringen Teil des Ansatzes (der Lösung) die Kapillaren am Auslaufende in konzentrierter Form verläßt. Der Braunstein kann anschließend vom Enzym, vorzugsweise durch Filtration abgetrennt werden. Das abgetrennte Enzym kann sofort erneut mit L-Arginin in gleicher Weise umgesetzt oder für eine spätere Anwendung aufbewahrt werden.

Es wird vermutet, daß diese ungewöhnlich hohe Stabilisierung u. a. dadurch zustande kommt, daß das Reduktionsmittel teilweise die Oxidation des Mn²⁺ zum Mn⁴⁺ unterbindet, wobei die Oxidation jedoch nicht vollständig unterbunden werden darf, da geringe Mengen an Mn⁴⁺ für eine gute Aktivität des Enzyms vermutlich wiederum günstig sind. Das heißt also, daß bei einem verhältnismäßig niedrigem pH, d. h. zwischen 8,5 und 10,5, das Reduktionsmittel gegenüber dem Mn^{2+/4+}-System im Unterschuß vorliegen sollte, damit die Oxidation des Mn²⁺ nicht vollständig unterbunden wird. Andererseits sind bei einem höheren pH, im vorliegenden Fall bis ca. 11,5, die bei Enzymreaktionen üblicherweise verwendbaren Reduktionsmittel nicht so stark, daß sie die Oxidation des Mn²⁺ vollständig verhindern können, es kann hier entsprechend auch ein Überschuß an Reduktionsmittel gegenüber dem Mn²⁺ eingesetzt werden. pH und Reduktionsmittel sollten also so aufeinander abgestimmt sein, daß ein langsamer Ausfall von Mangandioxid gewährleistet ist, da dieses vermutlich zum Aufbau einer Schutzschicht auf der Ultrafiltrationsmembrane führt, über die das Enzym von den Edukten und Produkten wieder abgetrennt werden kann. Die Ultrafiltrationsmembrane kann dabei in einem Enzymmembranreaktor oder auch als separate Einheit angeordnet sein und betrieben werden. Die Reduktion wird also vorteilhaft in Gegenwart von MnO₂ durchgeführt, das auch während der Reaktion gebildet werden kann.

Die für den Arginaseansatz notwendigen bzw. günstigen Komponenten sind einzeln und in Mischung oder Teilmischung so stabil, daß sie günstigerweise als Kit angeboten und gelagert werden können. Das heißt, ein Kit enthält aufeinander abgestimmte Mengen an Arginase und Reduktionsmittel. Vorteilhafterweise enthält der Kit auch Mn²⁺ als z. B. Mangansulfat oder Manganchlorid und/oder gegebenenfalls auch noch ein Substrat wie Arginin sowie ggf. auch L-Ornithin. L-Ornithin ist zwar ein kompetitiver Inhibitor des Enzyms, der aber als Stabilisator bei der Lagerung der Arginase (in Lösung und in lyophilisierter Form) dient. Zum Ansetzen des Ansatzes braucht der Kid dann nur noch in der entsprechenden Menge vorzugsweise sterilisiertem Wasser gelöst zu werden. Dem Kit kann ferner noch ein Teil der benötigten Gerätschaften, wie z. B. Kapillarultrafiltrationskartuschen beigegeben werden, insbesondere wenn es sich hierbei um Einmalprodukte handelt.

Die Erfindung wird im folgenden anhand von Beispielen und Zeichnungen weiter erläutert.

Es zeigen:
- Fig. 1: eine Ausführung eines Arginaseansatzes;
- Fig. 2: ein Verfahren zur enzymatischen Gewinnung von Ornithin aus Arginin.

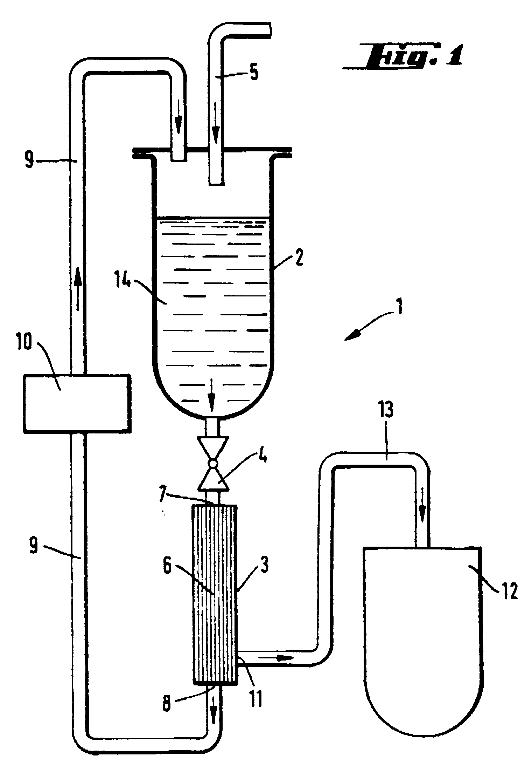

Der Batch-Ultrafiltrations-Reaktor 1 in Fig. 1 enthält ein Reaktionsgefäß 2 und eine Ultrafiltrations-Kartusche 3, die über ein Ventil 4 miteinander verbunden sind. Das Reaktionsgefäß 2 enthält einen Reaktionsansatz 14, ist verschließbar und an eine Druckleitung 5 angeschlossen. Nach beendeter Reaktion kann über die Druckleitung 5 und bei geöffnetem Ventil der Reaktionsansatz 14 ohne große Verwirbelung mittels N₂ durch die Kartusche 3 gepreßt werden. Die Kartusche 3 enthält eine Vielzahl poröser Kapillaren 6, deren eines Ende 7 über das Ventil 4 mit dem Reaktionsgefäß 2 verbunden ist und deren anderes Ende 8 an eine Rückführleitung 9 angeschlossen ist, über die das die Kapillaren passierte Enzym wieder in das Reaktionsgefäß 2 zurückgegeben werden kann. In der Rückführleitung 9 ist ein Filter 10 angeordnet zur Abtrennung des die Kartusche 3 passierten Braunsteins. Das Kapillarenäußere ist über einen Auslauf 11 mit einem Produktsammelbehälter 12 verbunden 13, in dem die niedermolekularen Bestandteile des Reaktionsansatzes 14 (Ornithin, ggf. nicht umgesetztes Arginin, Puffersalze, Reste an Reduktionsmittel und Mn²⁺) gesammelt werden.

Mit dem zurückgeführten Enzym kann erneut ein Arginaseansatz betrieben werden.

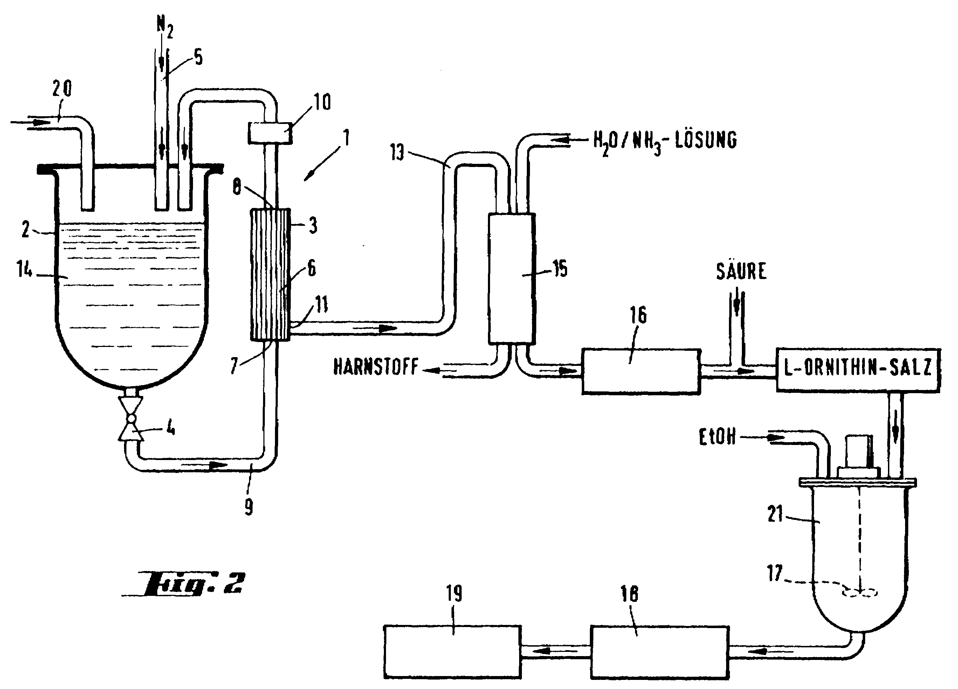

Das Verfahrenschema in Fig. 2 enthält den Batch-Ultrafiltrationsreaktor 1 analog Fig. 1, der Auslauf 11 der Kartusche 3 ist jedoch mit einer Kationenaustauschersäule 15 verbunden, auf der das Ornithin des Filtrats der Kartusche 3 zurückgehalten wird, wobei Harnstoff und Anionen die Säule passieren. Die mit Ornithin beladene Säule 15 wird gewaschen und mit Ammoniaklösung eluiert. Das eluierte Ornithin wird bis zur Sättigung der Lösung in einem Behälter 16 konzentriert, mit Säure (z. B. HCl, H₂SO₄, L-Aspartat, α-Ketoglutarsäure, Essigsäure) neutralisiert bis schwach sauer eingestellt, und in einem Fällungsbehälter 21 mit ca. der 3-fachen Menge an EtOH versetzt. Unter Rühren 17 fällt dabei das entsprechende Ornithinsalz aus, wird über einen Filter 18 abgetrennt und in einem Trockner 19 auf den gewünschten Trockengrad gebracht. Über eine verschließbare Öffnung 20 am Reaktionsgefäß 2 wird jeder neue Ansatz mit den entsprechenden Komponenten (verbrauchtes Enzym, H₂O, Reduktionsmittel usw.) ergänzt.

### Beispiel 1:

Zu 1 l einer 0.75 molaren Lösung von L-Arginin, die mit Schwefelsäure auf einen pH-Wert von 9,5 eingestellt worden ist, werden 2,5 × 10⁻⁴ Mol Mangansulfat × H₂O, ein halbes Äquivalent, also 1,25 × 10⁻⁴ Mol Ascorbinsäure, sowie 10.000 Units Kalbsleber-Arginase (Boehringer Mannheim, Deutschland) zugegeben. Nach 24 Stunden betrug der Umsatz an L-Arginin 98 %, nach weiteren 24 Stunden 100 %.

Nach Ultrafiltration über ein Hohlfasermodul (Fa. Amicon, MWCO 10,000, 0,03 m²) wurde das Filtrat über einen sauren Ionenaustauscher gegeben, mit ca. 1 l 5%-igem Ammoniak eluiert, auf 500 ml eingeengt, mit ca. 26 - 28 ml konzentrierter Schwefelsäure auf einen pH-Wert von 6,9 eingestellt und mit 1,5 l Ethanol das Ornithin-Sulfat ausgefällt. Es wurden 114,3 g reines Ornithin-Sulfat erhalten (84,6 % der Theorie).

### Beispiel 2:

Es wurde analog verfahren wie in Beispiel 1, nur daß eine L-Argininkonzentration von 0,5 mol/l und 6600 Units des Enzyms verwendet wurden. Es wurden 82,2 g L-Ornithin-Sulfat (91,3 % der Theorie) erhalten.

### Beispiel 3:

Es wurde analog verfahren wie in Beispiel 1, nur daß eine L-Argininkonzentration von 1 mol/l und 9400 Units der Kalbsleber-Arginase verwendet wurden. Die Ausbeute an L-Ornithin-Sulfat betrug 141,4 g (78,5 % der Theorie).

### Beispiel 4:

30 ml einer 0,75 molaren L-Argininlösung wurden mit 2,5 × 10⁻⁴ Mol Mangansulfat × H₂O, mit unterschiedlichen Mengen Ascorbinsäure versetzt und einmal beim Eigen-pH-Wert des Arginins (ca. pH 11,5) und einmal mit Salzsäure stabilisiert bei einem Anfangs-pH-Wert von 9,5 mit 370 Units Kalbsleber-Arginase versetzt. Nach 23 Stunden bei Raumtemperatur wurden die Umsätze an Arginin bestimmt. Anschließend wurde über ein Ultrafilter das Enzym abgetrennt und im nächsten Batchansatz erneut eingesetzt.

| **Anfangs-pH-Wert 9.5** | | | | |
|---|---|---|---|---|
| | blanke Membran | | blanke Membran | |
| Batch-Nr. | Umsatz (%) | Ascorbinsäure (mol/l) | Umsatz (%) | Ascorbinsäure (mol/l) |
| 1 | 52 | 2,5 x 10⁻⁴ | 67 | 1,25 x 10⁻⁴ |
| 2 | 37 | | 56 | " |
| 3 | 13 | | 41 | " |
| 4 | 7 | | 37 | " |
| 5 | 4 | | 30 | 0 * |
| 6 | 0 | | 29 | 0 * |
| 7 | - | | 29 | 0 * |
| 8 | - | | 28 | 0 * |

| | | | | |
|---|---|---|---|---|
| * = Ausfall von Braunstein | | | | |

| **Anfangs-pH-Wert 9,5** | | |
|---|---|---|
| Batch-Nr. | Braunstein-belegte Membran | |
| | Umsatz (%) | Ascorbinsäure (mol/l) |
| 1 | 59 | 2,5 x 10⁻⁴ |
| 2 | 31 | |
| 3 | 19 | |
| 4 | 16 | |
| 5 | 12 | |
| 6 | 10 | |
| 7 | 8 | |
| 8 | 7 | |

| Anfangs-pH-Wert 11,5 | | | | |
|---|---|---|---|---|
| Batch-Nr. | Braunstein-belegte Membran | | blanke Membran | |
| | Umsatz (%) | Ascorbinsäure (mol/l) | Umsatz (%) | Ascorbinsäure (mol/l) |
| 1 | 79 | 2,5 x 10⁻⁴ | 79 | 2,5 x 10⁻⁴ |
| 2 | 73 | | 73 | |
| 3 | 69 | | 72 | |
| 4 | 70 | | 65 | |
| 5 | 67 | | 61 | |
| 6 | 66 | | 61 | |
| 7 | 66 | | 62 | |
| 8 | 66 | | 61 | |

Bei einem Anfangs-pH-Wert von 9,5 desaktiviert das Enzym sowohl bei anfänglich Braunstein-belegter als auch bei blanker Membran, weil bei diesem pH-Wert und genügend Ascorbinsäure die Membran keine Braunstein-Belegung halten kann. Setzt man jedoch Ascorbinsäure gar nicht oder nur im Unterschuß zu, bildet sich eine Braunstein-Schicht und das Enzym desaktiviert nicht mehr stark. Bei pH 11,5 jedoch bildet sich schnell eine Braunstein-Schicht auf blanker Membran und das Enzym desaktiviert in jedem Fall wenig.

### Beispiel 5:

In einem Enzym-Membran-Kreislaufreaktor von 12 l Volumen wird eine 0,75 molare L-Argininlösung vorgelegt und 0,51 g Mangansulfat x H₂O (2,5 x 10⁻⁴ molar), 0,53 g bzw. 0,265 g Ascorbinsäure (2,5 bzw. 1,25 x 10⁻⁴ molar) sowie 60 000 Units (5000 Units/l) Kalbsleber-Arginase zugegeben und der pH-Wert mit Schwefelsäure auf 9,5 eingestellt. Nach 23h Reaktionszeit im nicht bewegten Kreislaufmedium wurde der Umsatz bestimmt, das Enzym mit Stickstoff-Druckförderung über ein 2,4 m²-Ultrafiltrations-Hohlfasermodul der Fa. Romicon abgetrennt und im nächsten Batch erneut der Reaktion zugeführt.

| **Anfangs-pH-Wert 9.5** | | | | |
|---|---|---|---|---|
| | blanke Membran | | blanke Membran | |
| Batch-Nr. | Umsatz (%) | Ascorbinsäure (mol/l) | Umsatz (%) | Ascorbinsäure (mol/l) |
| 1 | 70 | 2,5 x 10⁻⁴ | 88 | 1,25 x 10⁻⁴ |
| 2 | 50 | " | 86 | " |
| 3 | 35 | " | 90 | " |
| 4 | 20 | " | 84 | " |
| 5 | 4 | " | 84 | " |
| 6 | 1,5 | " | 84 | " |
| 7 | 0 | " | 83 | " |
| 8 | - | | 84 | " |

Bei äquimolarer Zugabe von Mangansulfat desaktivierte das Enzym schnell, während bei halbäquivalenter Zugabe sich bereits nach 23 Stunden leichter Braunstein-Ausfall bemerkbar machte und die Arginase nur wenig desaktivierte.

### Beispiel 6: Herstellung von L-Ornithin-Acetat

Zu 1 l einer 0,75 molaren L-Argininlösung wurden 42,3 mg Mangansulfat-Hydrat (2,5 x 10⁻⁴ molar), 21,8 mg Ascorbinsäure (1,25 x 10⁻⁴ molar) sowie 15000 Units Kalbsleber-Arginase der Fa. Boehringer Mannheim zugegeben. Nach 48 Stunden war der Umsatz laut HPLC 100 %-ig. Mit Essigsäure wurde auf pH 6,8 eingestellt, drei Viertel des Wassers abgezogen und mit einem Liter Ethanol bei Raumtemperatur das Ornithin-Acetat ausgefällt. Nach 15 min Nachrühren wurde der Niederschlag abfiltriert, mit Ethanol nachgewaschen und im Vakuum (40 mbar) getrocknet. Die Ausbeute an reinem Ornithin-Acetat betrug 137,0 g (95 % der Theorie). Drehwert (c = 5 in Wasser): + 10,0^{o} (Soll: + 9,0 bis + 11,0^{o}).

### Beispiel 7:

Arginase-Kit für 1 l Ansatz

| 10 000 Units Arginase | |
|---|---|
| 170 mg (1 mmol) | L-Ornithin × HCl |
| 42,3 mg (0,25 mmol) | Mangansulfat × H₂O |
| 43,7 mg (0,125 mmol) | L-Ascorbinsäure |
| 174,2 g (1 mol) | L-Arginin. |

Das Enzym (in lyophilisierter Form) und das L-Ornithin liegen in Mischung in einem Gefäß (Ampulle) vor, dem insbesondere auch das Mangansulfat und die Ascorbinsäure zugemischt sein können. Das Arginin wird normalerweise getrennt beigelegt, kann aber auch mit den übrigen Komponenten gemischt vorliegen.

Für den Ansatz wird alles in einen Liter Wasser gegeben und ca. 2 Tage stehengelassen.

## Patentansprüche

1. Arginaseansatz, enthaltend eine Arginase, Wasser und ein Substrat,
**gekennzeichnet durch**
Ascorbinsäure als Reduktionsmittel, gelöst in dem Wasser in einer gegenüber der Arginase mindestens 10-fachen molaren Konzentration.

2. Arginaseansatz nach Anspruch 1,
**dadurch gekennzeichnet**,
daß das Reduktionsmittel gegenüber der Arginase in einer 10 bis 10⁶-fachen molaren Konzentration vorliegt.

3. Arginaseansatz nach einem der vorhergehenden Ansprüche,
**dadurch gekennzeichnet,**
daß die Arginase in einer Konzentration von 10⁻⁸ bis 10⁻⁵ mol/l vorliegt.

4. Arginaseansatz nach einem der vorhergehenden Ansprüche,
**dadurch gekennzeichnet,**
daß das Substrat Arginin ist und in einer Menge von 0,1 bis 2,0 mol/l vorliegt, wobei gegebenenfalls ein Teil des Arginins ungelöst ist.

5. Arginaseansatz nach einem der vorhergehenden Ansprüche,
**gekennzeichnet durch**
Mn²⁺ in gegenüber der Arginase 10 bis 10⁶-fachem molaren Überschuß.

6. Arginaseansatz nach Anspruch 5,
**dadurch gekennzeichnet,**
daß das Reduktionsmittel gegenüber dem Mn²⁺ in stöchiometrischem Überschuß vorliegt.

7. Arginaseansatz, enthaltend in wässriger Lösung eine Arginase, ein Substrat und gegenüber der Arginase mindestens 10-facher molaren Konzentration Ascorbinsäure, sowie gegebenenfalls Mn²⁺ und gegebenenfalls ein zusätzliches Puffersystem.

8. Arginase-Kit, enthaltend Ascorbinsäure als Reduktionsmittel und eine Arginase sowie gegebenenfalls Mn²⁺ in Form eines Salzes und gegebenenfalls Arginin zur Herstellung des Arginaseansatzes nach einem der Ansprüche 1 bis 7.

9. Verfahren zur enzymatischen Gewinnung von Ornithin aus Arginin in wässriger Lösung mittels einer Arginase und gegebenenfalls Mn²⁺,
**dadurch gekennzeichnet,**
daß in der Lösung als Reduktionsmittel Ascorbinsäure in einer mindestens 10-fachen molaren Menge gegenüber der Arginase gelöst wird.

10. Verfahren nach Anspruch 9,
**dadurch gekennzeichnet,**
daß das Reduktionsmittel in einer Konzentration von 10⁻⁷ bis 10⁻¹ mol/l zugegeben wird.

11. Verfahren nach Anspruch 10,
**dadurch gekennzeichnet,**
daß der pH zu 8eginn der Arginasereaktion auf 10,5 bis 11,5 eingestellt und das Reduktionsmittel in einer Konzentration von mindestens 10⁻⁵ mol/l zugegeben wird.

12. Verfahren nach Anspruch 9 oder 10,
**dadurch gekennzeichnet,**
daß das Reduktionsmittel gegenüber dem Mn²⁺ im stöchiometrischen Unterschuß eingesetzt wird.

13. Verfahren nach einem der Ansprüche 9 bis 12,
**dadurch gekennzeichnet,**
daß nach der Reaktion mittels Ultrafiltration der Reaktionsansatz zumindest weitgehend in ein Filtrat, das die niedermolekularen gelösten Bestandteile enthält, und die Arginase mit ungelösten Bestandteilen des Reaktionsansatzes aufgetrennt wird.

14. Verfahren nach Anspruch 13,
**dadurch gekennzeichnet,**
daß die Arginase nach der Ultrafiltration von vorliegendem unlöslichen MnO₂ abgetrennt, vorzugsweise abgefiltert wird.

15. Verfahren nach Anspruch 13 oder 14,
**dadurch gekennzeichnet,**
daß man das Filtrat über einen sauren Ionenaustauscher gibt, diesen anschließend vorzugsweise mit Ammoniak eluiert, das Eluat neutralisiert, vorzugsweise mit Schwefelsäure auf einen pH von ca. 6,9 einstellt, einengt, vorzugsweise bis zur Sättigung, und das L-Ornithin als Salz mit Ethanol, vorzugsweise der ca. dreifachen Menge (v/v), ausfällt.

16. Verfahren zur enzymatischen Gewinnung von Ornithin aus Arginin wobei man in wässriger Lösung Arginin mittels einer Arginase gegebenenfalls in Anwesenheit von Mn²⁺ und gegebenenfalls einem Puffersystem zu Ornithin umsetzt,
**dadurch gekennzeichnet,**
daß in der Lösung Ascorbinsäure in einer mindestens 10-fachen molaren Menge gegenüber der Arginase gelöst vorliegt.

## Claims

1. Arginase batch containing an arginase, water and a substrate,
characterised by
ascorbic acid as a reducing agent, dissolved in the water in an at least 10 times molar concentration relative to the arginase.

2. Arginase batch according to claim 1,
characterised in that
the reducing agent is present in a 10 to 10⁶ times molar concentration relative to the arginase.

3. Arginase batch according to one of the preceding claims,
characterised in that
the arginase is present in a concentration of 10⁻⁸ to 10⁻⁵ mol/l.

4. Arginase batch according to one of the preceding claims,
characterised in that
the substrate is arginine and is present in a quantity of 0.1 to 2.0 mol/l, wherein a proportion of the arginine is optionally undissolved.

5. Arginase batch according to one of the preceding claims
characterised by
Mn²⁺ in a 10 to 10⁶ times molar excess relative to the arginase.

6. Arginase batch according to claim 5,
characterised in that
the reducing agent is present in a stoichiometric excess relative to the Mn²⁺.

7. Arginase batch containing in an aqueous solution an arginase, a substrate and an at least 10 times molar concentration of ascorbic acid relative to the arginase, optionally together with M²⁺ and optionally an additional buffer system.

8. Arginase kit containing ascorbic acid as reducing agent and an arginase optionally together with Mn²⁺ in the form of a salt and optionally arginine for the production of the arginase batch according to one of claims 1 to 7.

9. Process for the enzymatic isolation of ornithine from arginine in an aqueous solution by means of an arginase and optionally Mn²⁺,
characterised in that
ascorbic acid is dissolved in the solution in an at least 10 times molar quantity relative to the arginase.

10. Process according to claim 9,
characterised in that
the reducing agent is added in a concentration of 10⁻⁷ to 10⁻¹ mol/l.

11. Process according to claim 10,
characterised in that
the pH at the beginning of the arginase reaction is adjusted to 10.5 to 11.5 and the reducing agent is added in a concentration of at least 10⁻⁵ mol/l.

12. Process according to claim 9 or 10,
characterised in that
the reducing agent is used in a stoichiometric deficit relative to the Mn²⁺.

13. Process according to one of claims 9 to 12,
characterised in that
after the reaction, the reaction batch is at least largely separated by ultrafiltration into a filtrate, which contains the low molecular weight dissolved constituents, and the arginase with undissolved constituents of the reaction batch.

14. Process according to claim 13,
characterised in that
after the ultrafiltration, the arginase is separated, preferably by filtration, from the insoluble MnO₂ which is present.

15. Process according to claim 13 or 14,
characterised in that
the filtrate is passed through an acidic ion exchanger, which is then eluted preferably with ammonia, the eluate is neutralised, preferably adjusted to a pH of approx. 6.9 with sulfuric acid, is concentrated, preferably to saturation, and the L-ornithine is precipitated as a salt with ethanol, preferably in approx. 3 times the quantity (v/v).

16. Process for the enzymatic isolation of ornithine from arginine, wherein arginine is reacted in an aqueous solution by means of an arginase, optionally in the presence of Mn²⁺ and optionally a buffer system, to yield ornithine,
characterised in that
ascorbic acid is present dissolved in the solution in an at least 10 times molar quantity relative to the arginase.

## Revendications

1. Charge d'arginine contenant une arginase, de l'eau et un substrat,
caractérisée par
de l'acide ascorbique comme réducteur, dissous dans l'eau en une concentration au moins 10 fois molaire par rapport à l'arginase.

2. Charge d'arginase selon la revendication 1,
caractérisée en ce que
le réducteur se présente par rapport à l'arginase en une concentration de 1 à 10⁶ fois molaire.

3. Charge d'arginase selon l'une des revendications précédentes,
caractérisée en ce que
l'arginase se présente en une concentration de 10⁻⁸ à 10⁻⁵ mole/litre.

4. Charge d'arginase selon l'une des revendications précédentes,
caractérisée en ce que
le substrat est l'arginine et est présente en une quantité de 0,1 à 2,0 mole/litre, une partie de l'arginine étant le cas échéant non dissoute.

5. Charge d'arginase selon l'une des revendications précédentes,
caractérisée par
Mn²⁺ en un excès de 10 à 10⁶ fois molaire par rapport à l'arginase.

6. Charge d'arginase selon la revendication 5,
caractérisée en ce que
le réducteur se présente par rapport à Mn²⁺ en un excès stoechiométrique.

7. Charge d'arginase contenant en solution aqueuse une arginase, un substrat et par rapport à l'arginase une concentration au moins 10 fois molaire d'acide ascorbique, ainsi que le cas échéant Mn²⁺ et le cas échéant un système tampon supplémentaire.

8. Trousse d'arginase contenant de l'acide ascorbique comme réducteur et une arginase ainsi que le cas échéant Mn²⁺ sous la forme d'un sel et le cas échéant de l'arginine pour la production de la charge d'arginase selon l'une des revendications 1 à 7.

9. Procédé d'obtention enzymatique d'ornithine à partir d'arginine en solution aqueuse au moyen d'une arginase et le cas échéant de Mn²⁺,
caractérisé en ce qu'
on dissout dans la solution, comme réducteur, de l'acide ascorbique en une quantité au moins dix fois molaire par rapport à l'arginase.

10. Procédé selon la revendication 9,
caractérisé en ce qu'
on ajoute le réducteur en une concentration de 10⁻⁷ à 10⁻¹ mole/litre.

11. Procédé selon la revendication 10,
caractérisé en ce qu'
on règle le pH au début de la réaction de l'arginase à 10,5 à 11,5 et en ce qu'on ajoute le réducteur à une concentration de 10⁻⁵ mole/litre.

12. Procédé selon la revendication 9 ou 10,
caractérisé en ce qu'
on utilise le réducteur par rapport au Mn²⁺ en un déficit stoechiométrique.

13. Procédé selon l'une des revendications 9 à 12,
caractérisé en ce qu'
après la réaction au moyen d'une ultrafiltration on sépare la charge réactionnelle au moins dans une large mesure dans un filtrat, qui contient les composants à faible poids moléculaire à l'état dissous, et l'arginase avec les composants non dissous de la charge réactionnelle.

14. Procédé selon la revendication 13,
caractérisé en ce qu'
on sépare l'arginase après l'ultrafiltration d'avec le MnO₂ insoluble présent, de préférence en procédant par filtration.

15. Procédé selon la revendication 13 ou 14,
caractérisé en ce qu'
on introduit le filtrat sur un échangeur d'ions acide, en ce qu'ensuite on élue celui-ci de préférence avec de l'ammoniaque, en ce qu'on neutralise l'éluat, de préférence en ce qu'on le règle à un pH d'environ 6,9 avec de l'acide sulfurique, en ce qu'on concentre, de préférence jusqu'à saturation, et en ce qu'on précipite la L-ornithine sous forme de sel avec de l'éthanol, de préférence en une quantité environ triple (v/v).

16. Procédé d'obtention enzymatique d'ornithine à partir d'arginine dans lequel on fait réagir en solution aqueuse de l'arginine au moyen d'une arginase, le cas échéant en présence de Mn²⁺ et le cas échéant d'un système tampon, pour donner de l'ornithine,
caractérisé en ce qu'
on trouve dans la solution de l'acide ascorbique à l'état dissous, en une quantité au moins 10 fois molaire par rapport à l'arginase.
